# EUROPEAN PATENT APPLICATION

(11) **EP 1 972 262 A2**
(43) Date of publication of application: **24.09.2008**
(21) Application number: 08005516.3
(22) Date of filing: 25.03.2008
(51) Int. Cl.: A61B 1/05, A61B 8/12

(54) **Medical apparatus obtaining information indicative of internal state of an object based on interaction between ultrasound waves and light**

(30) Priority: 23.03.2007 JP 2007077654
(71) Applicant: OLYMPUS MEDICAL SYSTEMS CORP., Shibuya-ku Tokyo 151-0072 (JP)
(72) Inventor: Igarashi, Makoto, Tokyo 151-0072 (JP); Gono, Kazuhiro, Tokyo 151-0072 (JP); Yoshino, Masahiro, Tokyo 151-0072 (JP)
(74) Representative: von Hellfeld, Axel

(57) **Abstract**

In a medical apparatus (1), an ultrasound generator (22g) generates ultrasound waves to be radiated into the region, and an ultrasound radiating-direction changing member (22s) changes in the region a radiating direction of the ultrasound waves to be generated by the ultrasound generator. An ultrasound converging member (22h) converges in the region the ultrasound waves subjected to the change of the radiating direction. Light which is reachable to the region is emitted from a light source unit (3,4). A light transmitting/receiving member (21a,b) radiates light emitted from the light source unit and receives light reflected in a converged region of the ultrasound waves in the region. Based on the reflected light, information showing the internal state in a desired deeper region in the membrana mucosa of the body tissue of the object.

## Description

### CROSS REFERENCE TO RELATED APPLICATION

The patent application related to and incorporates by reference Japanese Patent Application No. 2007-077654 filed on March 23, 2007.

### Background of the Invention

### (The field of the Invention)

The present invention relates to a medical apparatus for observing the inside of an object, and in particular, to the medical apparatus that is able to obtain information from a region being examined within the body tissue of the object on the basis of the interaction made between ultrasound waves and light.

### (Related art)

Various types of medical modalities have been known. An optical imaging apparatus, which has been highlighted recently, and the conventionally used endoscope are among those medical modalities. Of these medical apparatuses, the endoscope has been widely used in the medical field as well as the industrial field. Especially, in the medical field, the endoscope is used to observe tissue in patient's body cavities and others and perform various treatments.

Meanwhile, medical modalities which obtain tomographic images of objects based on optical imaging are also known recently. The optical imaging technique includes various types of techniques such as optical CT (computed tomography), optical coherence CT, photo-acoustic method, and ultrasound modulated optical tomography. In the medical field, these kinds of medical imaging techniques have attracted attention in observing various symptoms in objects in terms of a simple and noninvasive manner. Additionally, a system in which an optical imaging apparatus and an endoscope are implemented in a combined manner has also been known nowadays.

The ultrasound modulated optical tomography is an imaging technique that uses the interaction between the ultrasound waves and the light. In an apparatus for performing this ultrasound modulated optical tomography, ultrasound waves and light are radiated into the biological body of an object to cause the light to pass an in-body region in which the radiated ultrasound waves are locally converged. The light is subjected to modulation and/or scattering when passing the region to generate an optical component in which the modulation and/or scattering is reflected. The optical component is acquired as characteristic information, on which data of tomographic images of the body inside can be obtained. By way of example, Japanese Patent Application Publication (Laid-open) No. 2000-88743 discloses an optical measurement apparatus which provides tomographic images inside of a body by using the ultrasound modulated optical tomography.

However, the optical measurement apparatus disclosed by the publication is silent about positioning the converged region of the ultrasound waves, the radiating direction of the light, and the direction along which a region being examined exists. Thus, when observing a desired deeper region of an object being examined, it may be insufficient to acquire necessary characteristic information, because of insufficient scanning at the desired deeper region.

### Summary of the Invention

The present invention has been made in consideration of the foregoing conventional situation, and an object of the present invention is to provide a medical apparatus that has the capability of reliably acquiring characteristic information from a desired deeper region in the membrana mucosa of the body tissue of an object.

In order to achieve the above object, as one mode, the present invention provides an endoscope equipped with an insertion tube insertable into an object, the insertion tube having a distal end. As the basic components according to the present invention, the endoscope comprises: an ultrasound generator equipped in the distal end of the endoscope and configured to generate ultrasound waves to be radiated to a region being examined which resides within the object; an ultrasound radiating-direction changing member equipped in the distal end of the insertion tube and configured to change in the region a radiating direction of the ultrasound waves to be generated by the ultrasound generator; an ultrasound converging member equipped in the distal end of the insertion tube and configured to converge in the region the ultrasound waves subjected to the change of the radiating direction; and a light transmitting/receiving member equipped in the distal end of the insertion tube and configured to radiate light to be given and to receive light reflected in a converged region of the ultrasound waves in the region.

As another mode of the present invention, there is provided a medical apparatus for observing an internal state of a region being examined which resides within body tissue of an object. The apparatus comprises: an ultrasound generator that generates ultrasound waves to be radiated into the region; an ultrasound radiating-direction changing member that changes in the region a radiating direction of the ultrasound waves to be generated by the ultrasound generator; an ultrasound converging member that converges in the region the ultrasound waves subjected to the change of the radiating direction; a light source unit that emits light to be reachable to the region; and a light transmitting/receiving member that radiates light emitted from the light source unit and receives light reflected in a converged region of the ultrasound waves in the region.

### Brief Description of the Drawings

In the accompanying drawings:
Fig. 1 is a block diagram showing the configuration of essential components of a biological observation system in which an endoscope is employed, which is according to a first embodiment of the present invention;
Fig. 2A outlines a section showing a view of the distal end of the endoscope when being viewed along the X-axis direction;
Figs. 2B and 2C explain the operations of a transducer supporting member, in cooperation with Fig. 2A;
Fig. 3 is an oblique view showing a detailed construction of the distal end of the endoscope;
Fig. 4 is a section outlining a first modification of the construction of the distal end of the endoscope shown in Fig. 1;
Fig. 5 is an oblique view showing a detailed construction of the distal shown in Fig. 4;
Fig. 6 is a section outlining a second modification of the construction of the distal end of the endoscope shown in Fig. 1;
Fig. 7 is an oblique view showing a detailed construction of the distal shown in Fig. 6;
Fig. 8 is a block diagram showing the configuration of essential components of a biological observation system in which an endoscope is employed, which is according to a second embodiment of the present invention;
Fig. 9 is an oblique view showing a detailed construction of the distal end of the endoscope;
Fig. 10 is a view explaining the arrangement of mirrors and a beam splitter disposed in the distal end of the endoscope;
Fig. 11 is a block diagram showing the configuration of essential components of a biological observation system in which an endoscope is employed, which is according to a third embodiment of the present invention; and
Fig. 12 is an oblique view showing a detailed construction of the distal end of the endoscope.

### Detailed Description of the Preferred Embodiments

With reference to the accompanying drawings, various embodiments of the present invention will now be described.

### (First embodiment)

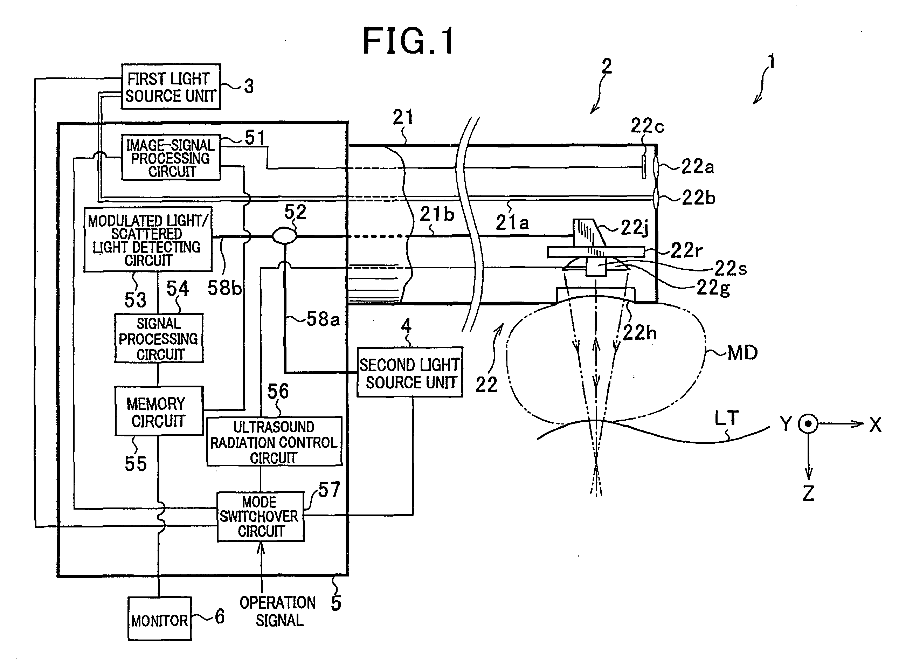

Referring to Figs. 1-3, a first embodiment of the present invention will now be described.

A biological observation system 1 according to the present embodiment is provided as the medical apparatus of the present invention. As shown in Fig. 1, the biological observation system 1 includes, as its essential components, an endoscope 2, a first light source unit 3, a second light source unit 4, a controller 5, and a monitor 6. Of these, the endoscope is insertable into a body cavity of an object being examined and is able to observe a region to be examined within a body tissue LT residing in the body cavity. The first light source unit 3 is configured to radiate illuminating light for illuminating the region to be examined in the conventional observation mode which is later described. The second light source unit 4 is configured to radiate light for observing the inside of the region to be examined in an ultrasound modulated optical tomography mode later described. The controller 5 is in charge of applying predetermined processes to electrical signals and light to be outputted from the endoscope 2 in order to produce image signals based on the electrical signals and light and output the produced image signals. The monitor 6 is configured to provide images depending on the image signals outputted from the controller 5.

The endoscope 2 is mechanically and electrically connectable with the controller 5, as shown in Fig. 1, and is equipped with an elongated and flexible insertion tube 21 which is insertable into a body cavity. This insertion tube 21 includes a cylindrical and rigid distal end 22 which serves as the distal end of the insertion tube 21. During the observation time, a space between the distal end 22 of the endoscope 2 and a body tissue LT (membrane mucosa, for example) is filled with a medium MD, such as water, to transmit ultrasound waves therethrough. Alternatively, the distal end 22 may be located to directly touch the body tissue LT without a gap therebetween.

In the present embodiment, as shown in Fig. 1, for the sake of convenience of the description, the XYZ orthogonal coordinate system is set such that the X-axis is assigned to the longitudinal direction (axis direction) of the distal end 22 of the endoscope 2.

In the insertion tube 21, there are provided various signal lines (not shown) to transmit electrical signals, a first light guide cable 21a, and a second light guide 21b. Of these, the first light guide cable 21a is used in the conventional observation mode to transmit, to the distal end 22, light emitted from the first light source unit 3 and dedicated to illuminate a desired region to be examined. The second light guide 21b is used, in the ultrasound modulated optical tomography mode, to not only transmit, to the distal end 22, light (radiating light) emitted from the second light source unit 4 and dedicated to being modulated at a desired region being examined but also transmit, to the controller 5, reflected light detected by the distal end 22.

In the distal end 22, there are provided an objective optical system 22a, an illuminating optical system 22b, and an image pickup device 22c. The objective optical system 22a and illuminating optical system 22b are arranged to have optical axes parallel with an inserting axis direction along which the insertion tube 21 is inserted (i.e., this inserting axis direction corresponds to the X-axis direction when the insertion tube 21 is inserted straight). The image pickup device 22c is located at a position where images captured by the objective optical system 22a are focused.

Under the conventional observation mode, the illuminating optical system 22b receives the light emitted from the first light source unit 3 and transmitted through the first light guide cable 21a and uses the light to illuminate a desired body tissue LT. The image pickup device 22c includes, as image pickup means, a CCD (charge-coupled device), for example. This mage pickup device 22c is used to image the body tissue LT under the conventional observation mode. This device 22c produces electrical signals in accordance with images of the body tissue LT focused by the objective optical system 22a and outputs the produced electrical signals to the controller 5.

In addition to the above components, the distal end 22 is equipped with an ultrasound transducer 22g, an acoustic lens 22h, a light reflection member 22j, a plate-like support member 22r, and a plurality of transducer supporting members 22s which slidably support the ultrasound transducer 22g. In the present embodiment, the plurality of transducer supporting members 22s are two in number, which are arranged in parallel in the Y-axis direction.

The ultrasound transducer 22g comprises piezoelectric elements serving as ultrasound generating members. This ultrasound transducer 22g is circular when viewed in its axial direction and bowl-shaped to have a concave area when viewed from a side direction, and the concave area includes a surface (ultrasound output area) to output ultrasound waves outward. This transducer 22g has also a rear surface which is back-to-back to the ultrasound output area. In reply to mechanical forces being applied, the curvature of the concave area of this ultrasound transducer 22g is changeable within a predetermined range.

Under the control of the controller 5, the ultrasound transducer 33g emits the ultrasound waves toward a body tissue LT of an object to be examined. The emitted ultrasound waves are propagated through the body tissue LT.

The acoustic lens 22h is opposed to the ultrasound output area of the ultrasound transducer 22g. The ultrasound waves emitted from the ultrasound transducer 22g passes this acoustic lens 22h, which allows the ultrasound waves to be converged (beam-formed) to produce a desired converged region.

The light reflection member 22j includes optical systems such as prisms, and works as a light transmitting/receiving member. This member 22j reflects the light coming via the second light guide 21b along the direction parallel with the inserting axis direction (the X-axis direction) of the endoscope 2, along the perpendicular direction (the Z-axis direction) to the inserting axis direction. In addition, this light reflection member 22j receives and reflects the light coming along the Z-axis direction to the second light guide 21b.

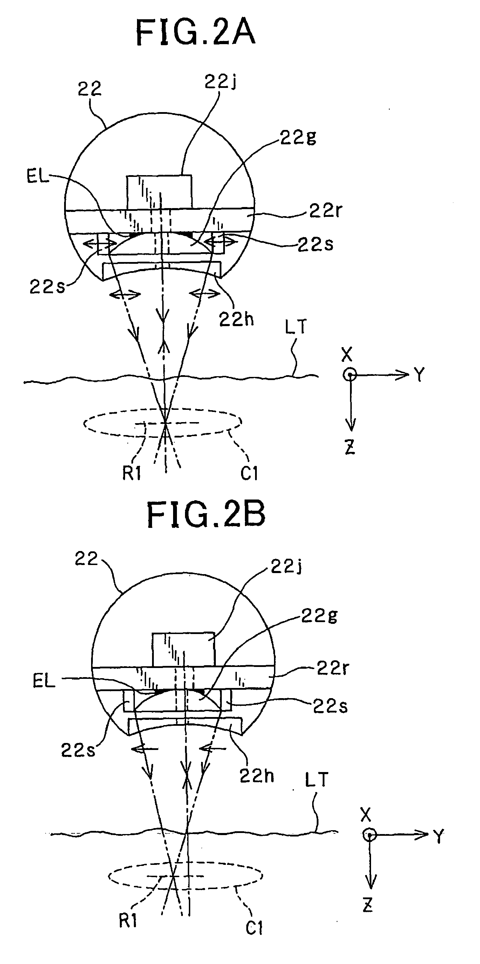

The support members 22r is located between the light reflection member 22j and the ultrasound transducer 22g. This member 22r supports the rear surface of the ultrasound transducer 22g via an elastic member EL secured to the rear surface of the ultrasound transducer 22g (refer to Figs. 2A-2C).

The support member 22r has not-shown grooves formed thereon in the Y-axis direction, and the grooves allow the respective transducer supporting members 22s to be slidable on the support member 22r, along the Y-axis direction as shown by arrows in Figs. 2A-2C. Further, this support member 22r has a through-hole formed therethrough at its center, as shown in Fig. 2A. Thus, the light transmitted to and from the light reflection member 22j along the Z-axis direction can pass the support member 22r without being blocked.

The plurality of transducer supporting members 22s work as an ultrasound radiating direction changing member. Each of the transducer supporting members 22s is arranged to touch the edge portion of the ultrasound transducer 22g to support this transducer 22g. Moreover, each of the transducer supporting members 22s is made to touch the support member 22r and to be slidable along each of the grooves formed on the support member 22r. Thus the transducer supporting members 22s are slidable individually in the planar direction of the support member 22r.

The sliding operations of the transducer supporting members 22s are controlled by a command from the controller 5. For the sliding operations, each of the plurality of transducer supporting members 22s has an actuator (not shown), such as a piezoelectric element, which receives a drive signal from the controller 5 and vibrates to enable the sliding operations.

The sliding operations causes the elastic member EL to deform elastically, which also deforms the ultrasound transducer 22g mechanically. Thus the curvature of the concave area of the ultrasound transducer 22g can be changed, so that the ultrasound output area can be changed in its shape. The directions of the ultrasound waves emitted from the ultrasound transducer 22g can therefore be changed, in a controlled manner, depending on the deformed shapes of the ultrasound output area of the transducer 22g.

Incidentally, not to block the light transmitted from and to the light reflection member 22j in the Z-axis direction, both the ultrasound transducer 22g and the acoustic lens 22h have through-holes formed therethrough at their centers, respectively. The through-hole formed through the acoustic lens 22h is filled with a transmissive material (not shown) such as a transmissive resin, so that the light cannot be blocked, but foreign maters can be blocked from coming into the distal end 22.

In the present embodiment, as described, the light reflection member 22j, which serves as the light transmitting/receiving member, has the light transmitting portion and the light receiving portion which are integrally formed. That is, the light transmitting portion transmits the light from the second light guide 21b to travel in the Z-axis direction and the light receiving portion receives the light reflected in the Z-axis direction to travel to the second light guide 21b. However, this is not a decisive structure, but the light transmitting and receiving portions may be produced as mutually separated devices.

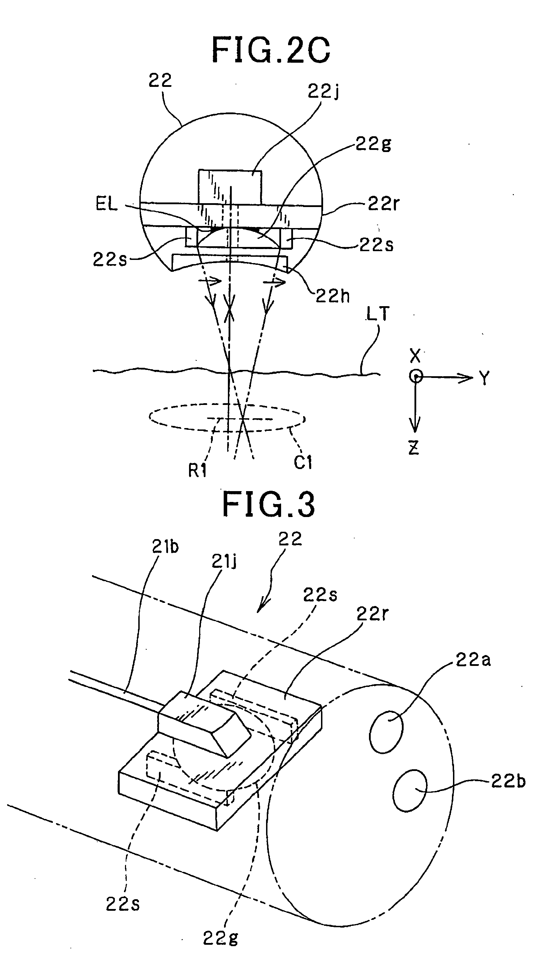

Fig. 3 shows a perspective view of the foregoing distal end 22, in which for the sake of an easier description, electrical wires and others are omitted from being depicted.

The ultrasound waves converged (beam-formed) by the acoustic lens 22h is propagated in the body tissue LT as a rarefactional wave which periodically oscillates in the longitudinal direction. In the body tissue LT, the ultrasound waves have a converged region of which sound pressure is denser. This converged region providing a denser sound pressure is able to function as an optical mirror which reflects (at least partly reflects) the light.

Hence the light emitted from the light reflection member 22j is reflected, at least partly reflected, by the denser sound-pressure region within the body tissue LT and returns, as reflected light, to the light reflection member 22j. The reflected light has been subjected to modulation (and/or scattering) in for example its frequency based on the interaction between the ultrasound waves and the light. From a viewpoint of the frequency, the frequency of the reflected light differs by Δf from that of the light before being radiated from the light reflection member 22j. This frequency shit is used to acquire information indicative of characteristics of the internal state of the object.

The first light source unit 3 has, for example, a xenon lamp which emits white light. This light source unit 3 is operative in the conventional observation mode and configured to emit the light, serving as illuminating light for illuminating an object to be observed, to the first light guide cable 21a under the control of the controller 5.

The second light source unit 4, which serves as a light source according to the present invention, emits light (i.e., light beam) that is able to reach a region to be examined residing within a body tissue LT. This second light source unit 4 is produced to have a laser light source or an SLD (Super Luminescent Diode), for example. The second light source unit 4 is for use in the ultrasound modulated optical tomography mode and responds to control from the controller 5 so as to emit the light (radiating light) to a light cable 58a. The radiated light passes the controller 5 by way of the light guide cable 58a later described.

The controller 5, serving as an image signal producer, includes an image-signal processing circuit 51, a light coupler 52, a modulated light/scattered light detecting circuit 53, a signal processing circuit 54, a memory circuit 55, a vibrating-member control circuit 56, a mode switchover circuit 57, and light guide cables 58a and 58b, as shown in Fig. 1.

The image-signal processing circuit 51 is under the control of the mode switchover circuit 57 and produces video signals depending on image pickup signals coming from the image pickup device 22c of the endoscope 2 and outputs the produced video signals to the memory circuit 55.

The light coupler 52 is optically connected to both the second light guide 21b and the light guide cables 58a and 58b arranged in the controller 5. This light coupler 52 receives light emitted from the second light source unit 4 and transmitted through the light guide cable 58a, and sends out the received light to the second light guide 21b. Meanwhile, reflected light coming through the second light guide 21b is sent to the modulated light/scattered light detecting circuit 53 via the light coupler 52 and the light guide cable 58b. In other words, the light coupler 52 functions as a light circulator.

The modulated light/scattered light detecting circuit 53 has a not-shown oscilloscope or a spectrum analyzer. This circuit 53 detects the reflected light (modulated light) coming via the light reflection member 22j, second light guide 21b, light coupler 52, and light guide cable 58b, and provides the signal processing unit 54 with an electrical modulated signal in accordance with the reflected light.

The signal processing circuit 54 receives the modulated signal from the detecting circuit 53 and estimates modulation characteristic information and/or scattering characteristic information (modulation/scattering characteristic information) of the light in a local region within the body tissue LT, the local region including a portion where the light is emitted from the light reflection member 22j. This signal processing circuit 54 produces video signals based on the estimated modulation/scattering characteristic information, and outputs the video signals to the memory circuit 55.

The memory circuit 55 temporarily memorizes either the video signals outputted from the image-signal processing circuit 51 in the conventional observation mode or the video signals outputted from the signal processing circuit 54 in the ultrasound modulated optical tomography mode. While memorizing the video signals, this circuit 55 also outputs the video signals to the monitor 6 sequentially, frame by frame.

The ultrasound radiation control circuit 56 is for controlling the radiated state of the ultrasound waves from the ultrasound transducer 22g in the ultrasound modulated optical tomography mode. Specifically, this control circuit 56 responds to operator's commands issued from a not-shown operation panel to control the slide operations of the transducer supporting members 22s. In reply to this control, as stated, the transducer supporting members 22s slides along the support member 22r, resulting in changes of the shape of the ultrasound output area of the ultrasound transducer 22g. That is, making the control circuit 56 control the transducer supporting member 22s leads to changes of an ultrasound converged region, that is, an ultrasound scanned region (i.e., position) in a region to be examined. This ultrasound converged region acts as an optically mirror that reflects (at least partly reflects) incident light into the region, because, within the body tissue LT, the ultrasound converged region has a denser sound pressure.

The mode switchover circuit 57 responds to a mode selection command manually given as an operation signal by an operator with a not-shown operation panel for the controller 5. In response to the command, the mode switchover circuit 57 selects, as the observation mode of the present biological observation system 1, either the conventional observation mode or the ultrasound modulated optical tomography mode.

Practically, when the observation mode is switched to the conventional observation mode in response to the operator's command, the mode switchover circuit 57 enables the operations of the first light source unit 3 and image-signal processing circuit 51 and disables the operations of the second light source unit 4, signal processing circuit 54, and ultrasound radiation control circuit 56. Thus in the conventional observation mode, the light emitted from the first light source unit 3 is used to illuminate the body tissue LT and the image pickup device 22c picks up the images of the body tissue LT.

In contrast, when the observation mode is switched to the ultrasound modulated optical tomography mode in response to the operator's command, the mode switchover circuit 57 enables the operations of the second light source unit 4, signal processing circuit 54, and ultrasound radiation control circuit 56. Concurrently the circuit 57 disables the operations of the first light source unit 3 and image-signal processing circuit 51. Thus in the ultrasound modulated optical tomography mode, the light (light beam) emitted from the light reflection member 22j and the ultrasound waves beam-formed by the acoustic lens 22h are radiated together to the body tissue LT.

There is a variation about the light radiated from the endoscope 2 into a region to be examined residing in the body tissue LT. In the foregoing, the second light source unit 4 is configured to emit the light converged previously, that is, the light beam, but this is just an example. Collecting lenses or other optical converging systems may be located in an optical path from the second light source unit 4 to the light reflection member 22j, so that the light emitted from this unit 4 is converged in the course of its travel.

The operations and advantages of the biological observation system 1 will now be described.

When the present system 1 is set to the conventional observation mode, the mode switchover circuit 57 enables the first light source unit 3 and the image-signal processing circuit 51 to be active as stated. Thus, the light from the first light source unit 3 travels through the first light guide cable 21a and is radiated toward a body tissue LT by the illuminating optical system 22b.

In the field of view of the objective optical system 22a, the image pickup device 22c picks up images of the body tissue LT to output image signals. Image signals are produced into video signals by the image-signal processing circuit 51, and the video signals are sent to the memory circuit 55.

The video signals from the processing circuit 51 are temporarily memorized by the memory circuit 55, during which the video signals are sent to the monitor frame by frame, in sequence. Therefore the monitor 6 uses the video signals to represent images of the body tissue LT for visual observation.

In contrast, when the observation mode of this system 1 is switched to the ultrasound modulated optical tomography mode, the second light source unit 4, signal processing circuit 54, and ultrasound radiation control circuit 56 are made active by the mode switchover circuit 57. Hence, the light from the second light source unit 4 travels along the light guide cable 58a, light coupler 52, and second light guide 21b, and is radiated toward the body tissue LT by the light reflection member 22j.

The ultrasound transducer 22g is controlled by the ultrasound radiation control circuit 56 such that the ultrasound waves are radiated toward the body tissue LT. During this radiation, by this control circuit 56, the two transducer supporting members 22s are driven to be slid along the Y-axis direction as shown in Figs. 2A, 2B and 2C, in response to an operator's command. The sliding motions of the transducer supporting members 22s allow the ultrasound output area of the ultrasound transducer 22g to be changed (distorted) in its shape in a controlled manner. Thus, the direction of the ultrasound waves to be emitted from the transducer 22g is changed depending on the changed shapes.

The ultrasound waves thus generated pass the acoustic lens 22h, so that the ultrasound waves are converged (beam-formed) in a converged region R1 in a region C1 to be examined residing the body tissue LT. Compared to a before-change position, the converged region R1 (i.e., converged position or scanned position) is positionally shifted by an amount depending on the change in the shape of the ultrasound output area of the ultrasound transducer 22g, as illustrated in Figs. 2A, 2B and 2C. This converged region R1 is the denser sound-pressure region, which serves as an optical mirror.

The light that has been radiated onto the denser sound-pressure region is subjected to specular reflection at positions where the refraction index changes. Hence at least part of the incident light is reflected by the denser sound-pressure region.

Accordingly, in the case of the embodiment, when the light is radiated onto the body tissue LT in the Z-axis direction, the reflection of light is caused by the ultrasound converged region, and the reflected light returns to the light reflection member 22j.

Concurrently, the ultrasound converged region R1 is moved in the body tissue LT in the Y-axis direction, as shown by Fig. 2A to 2B or 2A to 2B in response to the slide motions of the two transducer supporting member 22s that change the shape of the ultrasound output area of the ultrasound transducer 22g.

In Figs. 2A-2C, the ultrasound radiated from the ultrasound transducer 22g are depicted by chain double-dashed lines, while the light radiated by the light reflection member 22j is depicted by a dashed line. As shown in Fig. 2B or 2C, when the ultrasound radiating direction is changed within a given range in the lateral Y-axis direction, for example, to scan the ultrasound converge region R1, it is enough not to change the radiating direction of the light, because the ultrasound converged region has a certain degree of spread. Thus, with no change in the radiating direction of the light, the denser sound-pressure region is obtained in the spread and the reflected light is caused thereat.

The reflected light returned to the light reflection member 22j is then transmitted to the modulated light/scattered light detecting circuit 53 in the controller 5 via the second light guide 21b, the light coupler 52, and the light guide cable 58b.

In this detecting circuit 53, the reflected light is detected as an electrical modulated signal by oscilloscope processing and spectrum analyzing. The modulated signal is outputted to the signal processing circuit 54.

In the signal processing circuit 54, the modulated signal, which comes from the detecting circuit 53, is subjected to computation including Fourier transform to estimate modulation/scattering characteristic information in the ultrasound converged region and in the vicinity of this region, where the radiated light reflects. The estimated modulation/scattering characteristic information is then transformed into video signals, and the video signals are outputted to the memory circuit 55.

The memory circuit 55 temporarily holds the video signals, during which the video signals are outputted to the monitor 6 in sequence, frame by frame. Thus the video singles are displayed by the monitor 6 as tomographic images representing the estimated modulation/scattering characteristic information in the region C1 to be examined within the body tissue LT.

### (Modifications)

The distal end 22 is not always limited to the structure where, as shown in Figs. 2A-2C, the two transducer supporting members 22s are arranged in parallel in the Y-axis direction. For example, two transducer supporting members 22s may additionally be arranged in parallel in the X-axis direction, that is, the four transducer supporting members 22s are used in total.

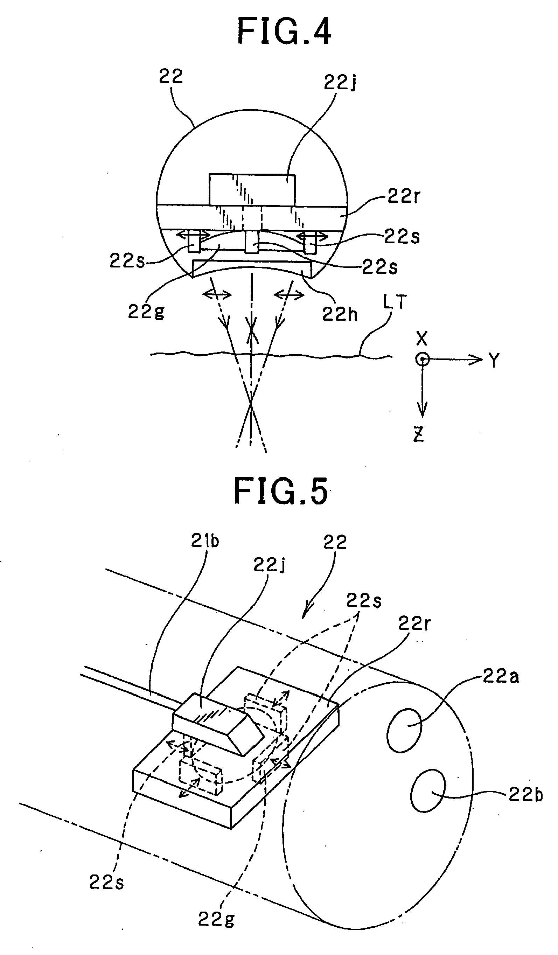

This example is shown in Figs. 4 and 5, where for the sake of a simplified description, electric wiring and others are omitted. As shown, the distal end 22 has four transducer supporting members 22s are provided in such a manner that the members 22s are suspended from not-shown grooves of the support member 22r and can be can be slid, pair by pair, along the support member 22r in each of the X- and X-axis directions. In other words, the two transducer supporting members 22s arranged in the Y-axis direction can be slid in the Y-axis direction, while, concurrently with or independently of this sliding motion, the two transducer supporting members 22s arranged in the X-axis direction can be slid in the X-axis direction.

Thus, when the ultrasound radiation control circuit 56 controls the drive of the four transducer supporting members 22s, it is possible that not only the direction of the ultrasound waves radiated from the ultrasound transducer 22g but alto their ultrasound converged region are changed two-dimensionally in the X- and Y-axis directions within the body tissue LT. Accordingly, the modulation/scattering characteristic information can be acquired from a wider region being examined in the body tissue LT.

Another modification will now be described.

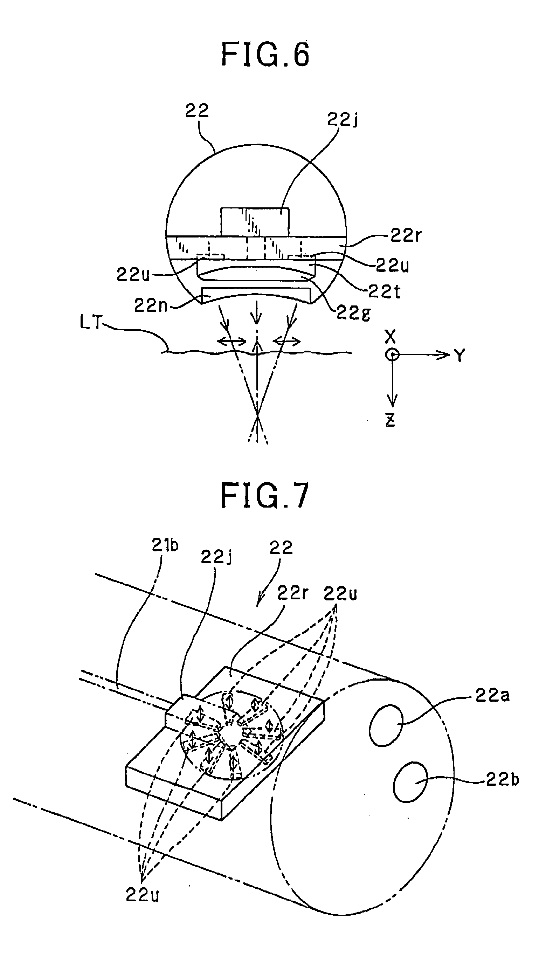

How to support the ultrasound transducer 22g for changing the shape of its ultrasound output area is not always limited to the exemplified in the first embodiment. Instead of using the foregoing transducer supporting members 22s, elastic members may be used. For example, the elastic members are placed on the rear surface of the ultrasound transducer 22g to as to be pushed/pulled for changing the shape of its ultrasound output area. This example is illustrated in Figs. 6 and 7.

As shown in Figs. 6 and 7, the distal end 22 differs from that explained in the first embodiment in that the transducer supporting members 22s are removed, while a plurality of elastic members 22t and a plurality of elastic-member deforming device 22u are additionally employed. The elastic members 22t are fixedly placed between the support member 22r and the rear surface of the ultrasound transducer 22g and the elastic-member deforming device 22u are arranged on the support member 22r.

The elastic members 22t are made of for example rubber and, using adhesive or other securing means, are made to adhere to the rear surface of the ultrasound transducer 22g, which is the opposite surface to the ultrasound output area thereof. Thus deformation of the elastic members 22t allows the ultrasound transducer 22g to be deformed as well.

The elastic-member deforming devices 22u are provided as the ultrasound radiating-direction changing member. Each of those deforming devices 22u is secured to each of the elastic members 22t, while each device 22u is moved (slid) vertically to the support member 22r along a not-shown vertical holes formed in the support member 22r. In addition, each of the elastic-member deforming devices 22u can be driven to move under the control of the ultrasound radiation control circuit 56, so that the elastic members 22t are pushed and/or pulled away from or to the support member 22r. Hence the elastic-member deforming devices 22u allows the elastic members 22t to change the shape of the ultrasound output area of the ultrasound transducer 22g.

In the example shown in Fig. 7, the elastic-member deforming devices are eight in number which are arranged at equal intervals, but this is not a decisive list. As long as it is sufficient to change the shape of the ultrasound output area, the number of deforming devices and the arranged positions thereof may be differentiated from the exemplified one.

In the present modification, the controller 5 controls the eight elastic-member deforming devices 22u, with the result that the radiating direction of the ultrasound waves and the denser sound-pressure region in the body tissue LT can be changed more finely within the region to be examined. This will lead to more accurate acquisition of the modulation/scattering characteristic information.

As stated, in the first embodiment and its modifications, the biological observation system 1 that uses the endoscope 2 is provided. This system 1 enables the ultrasound converged region to be changed (scanned) in the radiating direction of the light and in the vicinity of this light radiated direction. Thus without following the ultrasound converged region with the radiating light, information in the depth direction of the region being examined can be acquired efficiently. Compared to the conventional, it is therefore possible to acquire the characteristic information from a desired deeper region in the membrane mucosa of the body in a more reliable and positionally controlled manner.

### (Second embodiment)

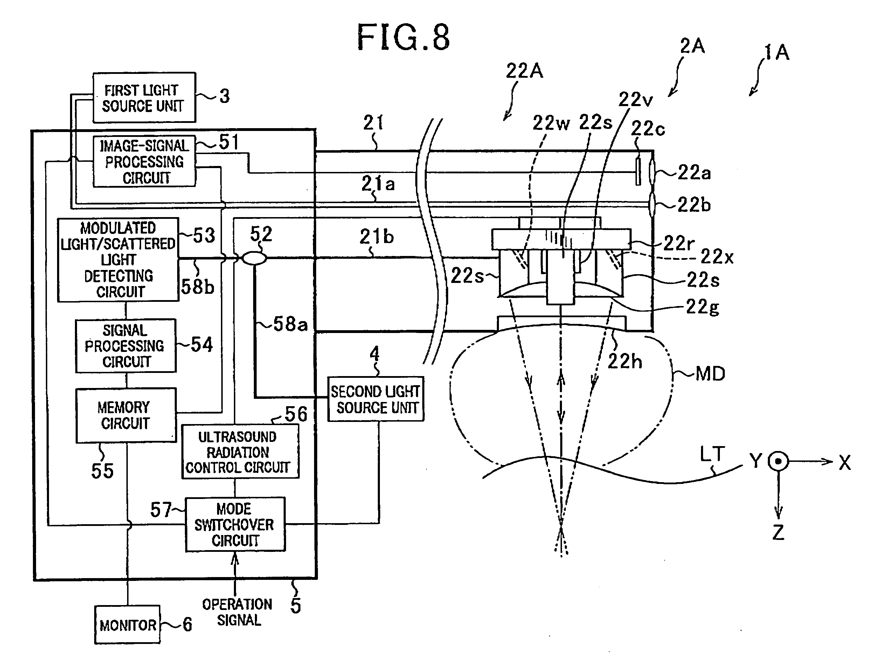

Referring to Figs. 8-10, a biological observation system according to a second embodiment of the present invention will now be described. In the second embodiment and succeeding embodiment, the components that are identical or similar to those in the first embodiment will be given the same reference numerals, for the sake of a simplified or omitted explanation.

Fig. 8 shows a biological observation system 1A of the present embodiment. This system 1A is provided with, as shown in Fig. 8, as its essential parts, an endoscope 2A which is similar in construction to the endoscope 2 of the first embodiment except for the distal end, the first and second light source units 3 and 4, the controller 5, and the monitor 6.

The endoscope 2A is equipped with an insertion tube 21 having the similar construction to that in the first embodiment and the insertion tube 21 comprises a distal end 22A positioned at the tip thereof. The distal end 22A is equipped with, like the first embodiment, the objective optical system 22a, the illuminating optical system 22b, and the image pickup device 22c.

Moreover, like the first embodiment, the distal end 22A is provided with the ultrasound transducer 22g, the acoustic lens 22h, the support member 22r, and a plurality of transducer supporting members 22s each attached to the edge of the ultrasound transducer 22g. The distal end 22A is additionally provided with a beam splitter 22v placed between the rear surface of the transducer 22g and the support member 22r.

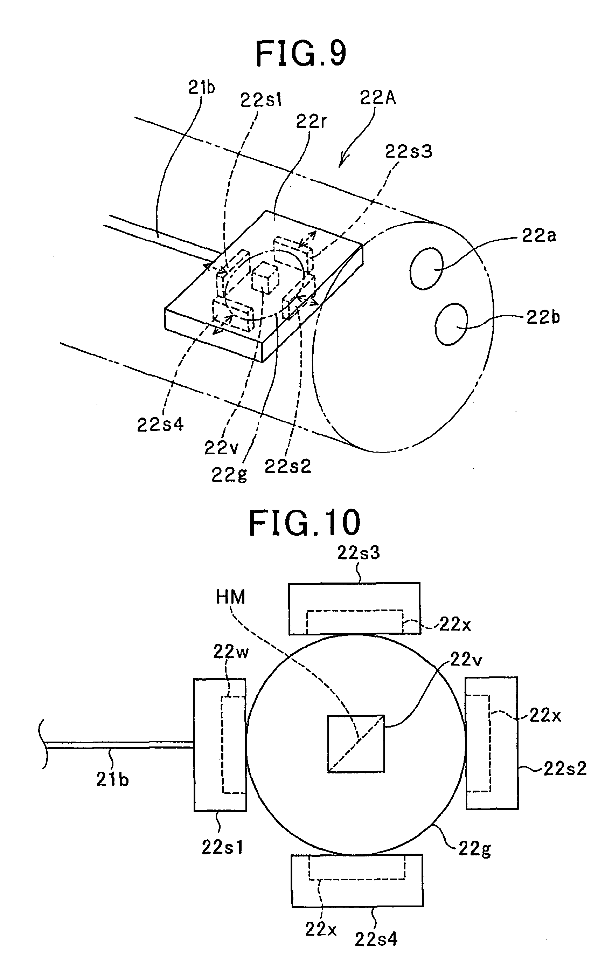

On the surface of the support member 22r, grooves (not show) are formed from which the transducer supporting members 22s are suspended and along which the members 22s are slid in the lateral XY plane (refer to arrows in Fig. 9). Differently from that in the first embodiment, the support member 22r of the present embodiment has no through-hole at the center thereof.

The transducer supporting members 22s include two transducer supporting members 22s (22s1, 22s2) arranged as a pair to be opposed to each other in the X-axis direction corresponding to the inserting axis direction of the endoscope 2A and two transducer supporting members 22s (22s3, 22s4) arranged as a pair to be opposed to each other in the Y-axis direction. That is, the four transducer supporting members 22s are arranged to support the edge of the ultrasound transducer 22g, as illustrated in Figs. 9 and 10.

The four transducer supporting members 22s serve as the ultrasound radiating-direction changing member and the light transmitting/receiving member. Of these four transducer supporting members 22, one of the members arranged in the X-axis direction, 22s1, contains a half mirror 22w optically connected to the second light guide 21b and arranged obliquely to the X- and Z-axis directions to have a predetermined oblique angle. The remaining member 22s2 paired to the above member 222s1 in the X-axis direction contains a total reflection mirror 22x arranged obliquely to the X-axis direction to have a predetermined oblique angle. That is, the transducer supporting members 22s1 and 22s2 serve as the light radiating-direction changing member.

The transducer supporting members 22s1 and 22s2 are made of optically trasnsmissive materials such as trasnsmissive plastic plate so as not to block the light passing therethrough.

Furthermore, of the four transducer supporting members 22s, each of the two transducer supporting members 22s3 and 22s4 arranged in the Y-axis direction contains a total reflection mirror 22x arranged obliquely to the Y- and Z-axis directions to have a predetermined oblique angle. Thus the transducer supporting members 22s3 and 22s4 serve as the light radiating-direction changing member.

Both the ultrasound transducer 22g and the acoustic lens 22h have the through-holes (not shown) as described before to pass the light. The not-shown through-hole of the acoustic lens 22h is filled with optically trasnsmissive material to allow the light pass therethrough and not to allow foreign matter to be blocked therein.

The beam splitter 22v contains a half mirror HM arranged to have predetermined angles oblique to the X- and Y-axis directions.

Incidentally Fig. 9 outlines the configuration of the distal end 22A, in which electrical wiring or others are omitted. When viewed along the Z-axis direction, the half mirror 22w, the total reflection mirror 22x, and the beam splitter 22v are arranged as shown in Fig. 10.

The controller 5 is the same as that in first embodiment.

The operations and advantages of the biological observation system 1A according to the present embodiment will now described.

In the conventional observation mode, under the control of the mode switchover circuit 57, the first light source unit 3 and the image-signal processing circuit 51 are made active, so that images of the surface of the body tissue LT are given, like the first embodiment.

In contrast, in the ultrasound modulated optical tomography mode, the mode switchover circuit 57 allows the second light source unit 4, the signal processing circuit 54, and the ultrasound radiation control circuit 56 to be active, instead of the foregoing. The light emitted from the second light source unit 4 enters the transducer supporting member 22s1 via the light guide cable 58a, the light coupler 52, and the second light guide 21b.

The light, which has entered the transducer supporting member 22s1, passes the half mirror 22w and travels to the beam splitter 22v. In the beam splitter 22v, the light is separated into two light beams; one light passes straight toward the transducer supporting member 22s and the other light reflects toward the transducer supporting member 22s3.

The light, which has entered the transducer supporting member 22s2, is reflected by the inner total reflection mirror 22x along the Z-axis direction. Meanwhile, the light, which has entered the transducer supporting member 22s3, is reflected by the inner total reflection mirror 22x along the Z-axis direction. Thus, the light is radiated onto the body tissue LT. The same is true of the transducer supporting member 22s4.

By making the ultrasound radiation control circuit 56 control the drive of the ultrasound transducer 22g as well as the transducer supporting members 22s1-22s4 arranged in the X- and Y-axis directions. Thus the ultrasound waves are radiated to the body tissue LT, during which the shape of the ultrasound output area thereof is changed in a controlled manner.

Hence the direction of the ultrasound waves emitted from the ultrasound transducer 22g is changed in accordance with the changed shape of its ultrasound output area, and the emitted ultrasound waves are converged by the acoustic lens 22h and propagated into the body tissue LT.

As described, the optical system, which is the half mirror 22w or the total reflection mirrors 22x, is contained in the transducer supporting members 22s1-22s4. Further, the transducer supporting members 22s are secured to the edge of the transducer 22g. Thus, the radiating direction of the light coming to the transducer supporting member 22s1 via the second light guide 21b follows the changes of the shape of the ultrasound output area of the ultrasound transducer 22g, that is, the changes of the radiating direction of the ultrasound waves from the ultrasound transducer 22g.

The ultrasound waves propagated in the body tissue produces its converged area due to the acoustic lens 22h. The converged region provides a denser sound pressure, which at least partly reflects the light passing therethrough. The reflected light thereat returns to for example the transducer supporting members 22s1 and 22s3.

The reflected light returned to the transducer supporting member 22s1 is reflected by its half mirror 22w, and then sent to the second light guide 21b. Meanwhile, the reflected light returned to the transducer supporting member 22s3 is sent to the second light guide 21b via the total reflection mirror 22x of the support member 22s4, the beam splitter 22v, and the half mirror 22w of the support member 22s1. The light is thus transmitted through the second light guide 21b to the modulated light/scattered light detecting circuit 53 in controller 5 via the light coupler 52 and the light guide cable 58b.

The remaining operations of the controller 5 are the same as those in the first embodiment. Hence, depending on the modulation/scattering characteristic information estimated by the signal processing circuit 54, tomographic images of the scanned ultrasound converged region within the tissue body LT are presented by the monitor 6.

As described, in the biological observation system 1A according to the present embodiment, there can be provided the similar operations and advantages to those in the first embodiment. In addition, the light and the ultrasound waves can be synchronized with each other in changes of the radiated reactions. That is, the radiating direction of the light follows that of the ultrasound waves. Thus, the characteristic information can be acquired from a wider area in a desired deeper region in the membrane mucosa of the body in a more reliable and accurate manner.

### (Third embodiment)

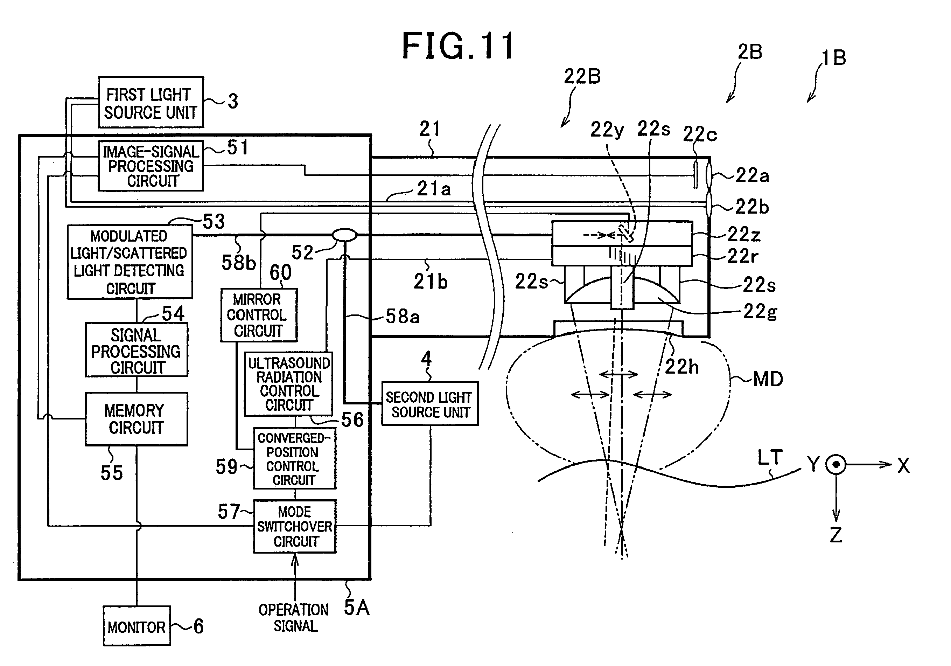

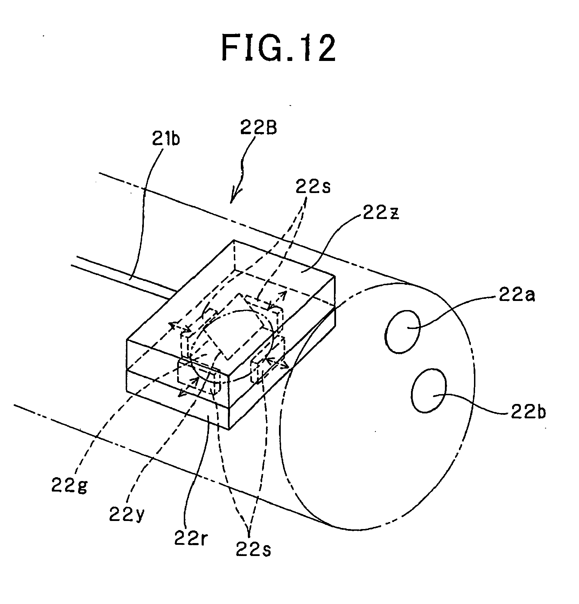

Referring to Figs. 11 and 12, a biological observation system according to a third embodiment of the present invention will now be described.

As shown in Fig. 11, a biological observation system 1B according to the present embodiment comprises, as its essential parts, an endoscope 2B characteristic of the present embodiment and a controller 5A which is in charge of controlling the operations of the endoscope 2B and producing video signals on information acquired by using the endoscope 2B, in addition to the first and second light source units 3 and 4 and the monitor 6.

The endoscope 2B is equipped with an insertion tube 21 mechanically and electrically connectable to the controller 5A and the insertion tube 21 includes an endoscope 22B located at the tip thereof, as illustrated in Fig. 11.

The objective optical system 22a, the illuminating optical system 22b, and the image pickup device 22c are also provided in the distal end 22B, like the foregoing.

Additionally, the distal end 22B is equipped with a mirror sustainer 22z with a scanning mirror 22y therein, in addition to the ultrasound transducer 22g, the acoustic lens 22h, the support member 22r, and the four transducer supporting members 22s. Like the foregoing, the transducer supporting members 22s supports the edge of the ultrasound transducer 22g using elastic members (not shown).

Specifically, the four transducer supporting members 22s include the two transducer supporting members 22s arranged to be opposed in the X-axis direction and each elastic member connects each member 22s and the edge of the transducer 22g. Likewise, the transducer supporting members 22s include the two transducer supporting members 22s arranged to be opposed in the Y-axis direction and each elastic member connects each member 22s and the edge of the transducer 22g.

The scanning mirror 22y functions as the light radiating-direction changing member. This scanning mirror 22y is connected to the controller 5A via a signal line when the endoscope 2B is connected to the controller 5A. The scanning mirror 22y is arranged in the mirror sustainer 22z to have oblique angles to the X-axis and Z-axis directions. The angles of the scanning mirror 22y can be controlled under the control of the controller 5A.

The mirror sustainer 22z functions as the light transmitting/receiving member. That is, this sustainer 22z allows the scanning mirror 22y to reflects the light coming from the second light guide 21b along the X-axis direction such that the reflected light travels along the Z-axis direction. In addition, the sustainer 22z allows the scanning mirror 22y reflects the light incoming along the Z-axis direction such that the reflected light travels to the second light guide 21b. The mirror sustainer 22z is made of optical trasnsmissive materials such as trasnsmissive plastic so as not to block the light passing therethrough.

The controller 5A is equipped with, in addition to the construction explained in the first embodiment, a converged-potion control circuit 59 and a mirror control circuit 60. The converged-position control circuit 59 controls both the ultrasound radiation control circuit 56 and the mirror control circuit 60 so that, under the control of the mode switchover circuit 57, the radiating directions of both the ultrasound waves and the light are made to agree with each other in the ultrasound modulated optical tomography mode.

Responsively to control of the converged-position control circuit 59, the ultrasound radiation control circuit 56 controls the drive of each of the plural transducer supporting members 22s, whereby the ultrasound waves radiated by the ultrasound transducer 22g can be transmitted in a desired direction.

Further, the mirror control circuit 60 responds to control of the converged-position control circuit 59 by driving the scanning mirror 22y such that the radiating direction of the light outgoing the mirror sustainer 22z for the radiation agrees with that of the ultrasound waves to be emitted in a controlled manner.

The operations and advantages of the biological observation system 1B will now be described.

In the conventional observation mode, the control of the mode switchover circuit 57 permits only the first light source unit 3 and the image-signal processing circuit 51 to be active. Thus, like the foregoing, the body tissue LT can be observed using images captured in the mode.

In contrast, in the ultrasound modulated optical tomography mode, the mode switchover circuit 57 allows only the second light source unit 4, the signal processing circuit 54, and the converged-position control circuit 59 to be active. The light emitted form the second light source unit 4 enters the mirror sustainer 22z via the light guide cable 58a, the light coupler 52, and the second light guide 21b. Concurrently with this, under the control of the converged-position control circuit 59, both the ultrasound radiation control circuit 56 and the mirror control circuit 60 operates as well.

Under the control of the ultrasound radiation control circuit 56, the ultrasound transducer 22g generates ultrasound waves radiated to the body tissue LT. In addition to this, this control circuit 56 responds to the control of the converged-position control circuit 59 by controlling the drive of the plural transducer supporting members 22s. This control changes the shape of the ultrasound output area of the ultrasound transducer 22g, thus allowing the generated ultrasound waves to be directed depending on the shape changes of the ultrasound output area. The ultrasound waves, of which direction is controlled by the shape changes, is converged by the acoustic lens 22h, and propagated within the body tissue LT.

In parallel, by responding the control of the converged-position control circuit 59, the mirror control circuit 60 drives the scanning mirror 22y such that the radiating direction of the light outgoing from the mirror sustainer 22z for the radiation agrees with the radiating direction of the ultrasound waves from the ultrasound transducer 22g.

Thus the light from the mirror sustainer 22z and the ultrasound waves through the acoustic lens 22h travel together toward the body tissue LT in almost the Z-axis direction.

The light propagated in the body tissue LT is reflected at an ultrasound converged region where the sound pressure is denser, whereby the reflected light returns to the mirror sustainer 22z.

The reflected light is reflected by the scanning mirror 22y in the mirror sustainer 22z such that the reflected light is transmitted to the modulated light/scattered light detecting circuit 53 in the controller 5A via the second light guide 21b, the light coupler 52, and the light guide cable 58b. In the controller 5A, the processes which are the same as the foregoing are applied to the reflected light.

Accordingly, in the ultrasound modulated optical tomography mode, the monitor 6 is able to represent tomographic images of the region being examined which are based on modulation/scattering characteristic information estimated by the signal processing circuit 54.

Therefore, in the biological observation system 1B of the present embodiment, the advantages similar to those gained in the second embodiment can be obtained. That is, by synchronizing the operations of the scanning mirror and the ultrasound transducer with each other, the direction of the radiated light and the converged region of the radiated ultrasound waves are positionally matched with each other, increasing reliability and accuracy in acquiring the characteristic information.

Although the descriptions above contain many specificities, these should not be construed as limiting the scope of the invention but as merely providing illustrations of some of the presently preferred embodiments of the present invention. Thus the scope of the present invention should be determined by the appended claims.

## Claims

1. An endoscope equipped with an insertion tube insertable into an object, the insertion tube having a distal end, the endoscope comprising:
an ultrasound generator equipped in the distal end of the endoscope and configured to generate ultrasound waves to be radiated to a region being examined which resides within the object;
an ultrasound radiating-direction changing member equipped in the distal end of the insertion tube and configured to change in the region a radiating direction of the ultrasound waves to be generated by the ultrasound generator;
an ultrasound converging member equipped in the distal end of the insertion tube and configured to converge in the region the ultrasound waves subjected to the change of the radiating direction; and
a light transmitting/receiving member equipped in the distal end of the insertion tube and configured to radiate light to be given and to receive light reflected in a converged region of the ultrasound waves in the region.

2. The endoscope of claim 1, wherein
the ultrasound radiating-direction changing member includes a member that mechanically deforms the ultrasound generator so that the ultrasound waves to be generated therefrom is changed in the radiating direction thereof.

3. The endoscope of claim 1, comprising
a light radiating-direction changing member equipped in the distal end of the endoscope and configured to make a radiating direction of the light radiated from the light transmitting/receiving member toward the region agree with the radiating direction of the ultrasound wages.

4. The endoscope of claim 3, wherein the light radiating-direction changing member and the ultrasound radiating-direction changing member are unified with each other.

5. The endoscope of claim 1, wherein
the ultrasound generator is configured to generate the ultrasound waves in a direction perpendicular to a longitudinal direction of the distal end of the insertion tube, and
the light transmitting/receiving member is configured to transmit the light in the perpendicular to the longitudinal direction of the distal end.

6. The endoscope of claim 5, comprising a support member that fixedly supports the light transmitting/receiving member, movably supports the ultrasound radiating-direction changing member, and supports the ultrasound generator with elastic members placed between the elastic members and the supporting member,
wherein the ultrasound radiating-direction changing member is arranged to allow the generator to be movable in relation to the support member.

7. The endoscope of claim 6, wherein the ultrasound radiating-direction changing member supports an edge of the ultrasound generator.

8. A medical apparatus for observing an internal state of a region being examined which resides within body tissue of an object, the apparatus comprising:
an ultrasound generator that generates ultrasound waves to be radiated into the region;
an ultrasound radiating-direction changing member that changes in the region a radiating direction of the ultrasound waves to be generated by the ultrasound generator;
an ultrasound converging member that converges in the region the ultrasound waves subjected to the change of the radiating direction;
a light source unit that emits light to be reachable to the region; and
a light transmitting/receiving member that radiates light emitted from the light source unit and receives light reflected in a converged region of the ultrasound waves in the region.

9. The medical apparatus of claim 8, wherein
the ultrasound radiating-direction changing member includes a member that mechanically deforms the ultrasound generator so that the ultrasound waves to be generated therefrom is changed in the radiating direction thereof.

10. The medical apparatus of claim 8, comprising
a light radiating-direction changing member that makes a radiating direction of the light radiated from the light transmitting/receiving member toward the region agree with the radiating direction of the ultrasound wages.

11. The medical apparatus of claim 10, wherein the light radiating-direction changing member and the ultrasound radiating-direction changing member are unified with each other.

12. The medical apparatus of claim 8, wherein
the ultrasound generator is configured to generate the ultrasound waves in a direction perpendicular to a longitudinal direction of the distal end of the insertion tube, and
the light transmitting/receiving member is configured to transmit the light in the perpendicular to the longitudinal direction of the distal end.

13. The medical apparatus of claim 12, comprising a support member that fixedly supports the light transmitting/receiving member, movably supports the ultrasound radiating-direction changing member, and supports the ultrasound generator with elastic members placed between the elastic members and the supporting member,
wherein the ultrasound radiating-direction changing member is arranged to allow the generator to be movable in relation to the support member.

14. The medical apparatus of claim 13, wherein the ultrasound radiating-direction changing member supports an edge of the ultrasound generator.

15. The medical apparatus of claim 8, comprising an image signal producer that estimates, from the reflected light coming from the region, characteristic information indicative of at least one of modulation and scattering of the reflected light and produces image signals based on the characteristic information.

16. The medical apparatus of claim 15, comprising a display that displays images of the region depending on the image signals.
